# EUROPEAN PATENT SPECIFICATION

(11) **EP 0 406 363 B1**
(45) Date of publication and mention of the grant of the patent: **10.03.1993**
(21) Application number: 90900308.9
(22) Date of filing: 21.12.1989
(51) Int. Cl.: A61K 49/02, A61K 43/00

(54) **METHOD TO DIAGNOSE AMYLOIDOSES SPECIFICALLY AND SUBSTANCE THEREFOR**
VERFAHREN ZUR SPEZIFISCHEN DIAGNOSE VON AMYLOIDOSEN SOWIE DIE DAZUGEHÖRIGE SUBSTANZ
PROCEDE PERMETTANT DE DIAGNOSTIQUER SPECIFIQUEMENT LES AMYLOIDOSES ET SUBSTANCE UTILISEE A CET EFFET

(30) Priority: 21.12.1988 GB 8829862
(43) Date of publication of application: 09.01.1991
(73) Proprietor: AKZO N.V., NL-6824 BM Arnhem (NL)
(72) Inventor: SHALDON, Stanley, F-34080 Montpellier (FR); FLÖGE, Jürgen, D-3000 Hannover (DE); KOCH, Karl, Martin, D-3000 Hannover (DE)
(74) Representative: Sturt, Clifford Mark
(86) International application number: GB8901528
(87) International publication number: WO9006777

(56) References cited:
- THE LANCET, vol. 1, 25 June 1988, London (GB); P.N.HAWKINS et al., pp. 1413-1418*
- J. EXP. MED., vol. 167, no. 3, March 1988, The Rockefeller University Press, New York, NY (US); P.N.HAWKINS et al., pp. 903-913*
- CONTR. NEPHROL., vol. 61, 26 February 1988, Karger, Basel (CH); J.FLÖGE et al., pp. 27-36*

## Description

Amyloidosis is a disease entity caused by the localized or systemic deposition of certain intact or proteolytically cleaved amyloidogenic proteins in tissue. which may have life-threatening consequences. Since its first desception by Virchow in 1851 the definitive diagnosis of amyloidosis for a long time has been from postmortem diagnoses. In the middle of this century improved biopsy techniques allowed the diagnosis to be obtained at lifetime, but still nowadays the diagnoses of amyloidosis usually necessitates invasive procedures. A non-invasive procedure is described in an article by Hawkins, Myers, Lavender and Pepys: Lancet 1988; 1:1413-1418. However, the procedure is non-specific and can not differentiate between progressive and non-progressive deposits.

Therefore, little is known about the true clinical incidence/extent of amyloid deposition, early stages of amyloidosis or the natural courses of amyloidosis. The early diagnosis of amyloidosis is of crucial importance. as increasing evidence suggests. that at least two of the more common forms, AA- and AL-amyloidosis, can be retarded, halted or even reversed in their clincial course by appropriate therapy (Berglund K, Keller C, Thysell H, Alkylating cytostatic treatment in renal amyloidosis secondary to rheumatic disease. Ann Rheum Dis 1987; 46; 757-762. (Benson MD. Treatment of AL amyloidosis with melphalan. prednison and colchicine. Arthritis Rheum 1986; 29:683-687) Initial reports also suggest, that the clincial course of a new type of amyloid, AB2M-amyloidosis, a almost universal complication of chronic hemodialysis. can be improved by medical therapy. However. due to the problems inherent to lifetime diagnosis and especially to quantification of amyloid it cuuently is very difficult to objectively demonstrate the success or failure of a certain therapeutic regime.

The present invention is predicated on a method for precisely diagnosing and quantitating the mass of an individual fibrillar amyloid protein in a non-invasive way, in contrast to cuuently used invasive procedures (i.e. biopsies) or previously proposed non-invasive. but also non-specific procedure. This involves the use of a radiodiagnostic test. using specific labelled amyloidogenic proteins and their high affinity to the conesponding deposited amyloid. to visualize and quantify amyloid deposition during lifetime in a non-invasive manner.

By way of example. a description will now be given of how the invention may be used to diagnose and quantify the presence of AB2M-amyloid (also termed AB-amyloid or dialysis related/associated amyloid);
AB2M-amyloidisos has first been described in long-term hemodialysis patients, but it may occur with other renal replacementy therapies as well. The incidence of this amyloidosis reaches almost 100% in patients dialyzed for 15 years or more. Symptomatology includes carpal tunnel syndrome, pathological fractures due to bone cysts. destructive arthropathies. spondylarthropathies with severe neurologic consequences, intestinal bleeding, cardiac failure etc. (Floge J, Granolleras C, Shaldon S Koch KM "Dialysis-associated amyloidosis and β2-microglobulin". Contr Nephrology 1988; 61: 27-36). β2-microglobulin and proteolytically cleaved fragments derived thereof have been identified as the major constituents of the amyloid fibrills. β2-microglobulin is retained in renal failure and standard hemodialysis membranes offer no removal capacity for β2-microglobulin. It has been suggested. that certain dialysis membranes. which offer removal of β2-microglobulin, may decrease the incidence of amyloid deposition, but all studies so far had to rely on clinical symptomatology as an indicator of the presence of amyloid. The problem of finding the true incidence and extent of the amyloid deposition has hitherto prevented a definitive study on the effects of various forms of treatment such as high flux hemofiltration or renal transplantation on AB2M-amyloidosis.

Besides β2-microglobulin AB2M amyloid also contains amyloid P-component which is derived from serum amyloid P component (SAP). Amyloid P-component however is not specific for this type of amyloid. as it is bound to nearly all forms of amyloid by calcium-dependent binding to specific ligands. which seems to be present on almost every type of amyloid fibrill. It is a minor constituent of amyloid deposits, the mass present being in the range of 5-15% of the mass of fibrills present. Although it is intimately associated with the fibrills it is not part of their structure. The above mentioned non-invasive procedure is based upon a radiodiagnostic test using ¹²³I-labelled SAP - hence the non-specific nature of the procedure.

In accordance with the present invention, use is made of the specific radiolabelled amyloidogenic precursor-protein, which after enzymatic or physical change incorporates with the amyloid fibrills. Thus, using the present invention, the majority of the amyloid fibrillar mass can be detected and quantified using computerised scanning techniques. Furthermore. the invention also renders the detection system highly specific and can be expected to increase its sensitivity markedly.

Methodological aspects: β2-microglobulin was prepared from sterile hemofiltrate of a uremic patient, which was HIV-l-. HBsAg- and CMV-(IGM) negative and which had normal serum levels of liver-enzymes. Preparation and purification of β2-microglobulin was performed by lyophilisation, dialysis against 20mM Tris-HCL pH 8.5, followed by ion-exchange chromatography (DEAE-Sephacel®) and repeated gel-chromatography (Sephacryl® S200). The obtained protein was > 99% pure, as shown by SDS-PAGE electrophoresis, immunoblotting and HPLC. 0.25mg of purified β2-microglobulin were labelled with 500 MBq of Na⁻¹²³iodine (New England Nuclear) using the lactoperoxidase method (Enzymobead ™ Radioiodination Reagent. Biorad. München). Unbound radioactivity was removed by gel chromatography on a Sephade® G50 column equilibrated with sterile. pyrogenfree PBS-buffer. The fraction containing the labelled β2-microglobulin was checked for purity by HPLC. The iodinated β2-microglobulin was diluted in sterile, pyrogenfree PBS, containing 5% human serum albumin (Behringwerke. Marburg) to prevent radiation damage. sterilized by passage through a 0.22µm Millipore filter and finally tested for pyrogenicity (LAL-test and rabbit injection). TCA-precipitability and the ability of labelled β2-microglobulin to bind to anti-β2-microglobulin antibodies was tested in the last step.

In comparison to normal persons. long-term hemodialysis patients were examined, all of which have clinical evidence of amyloid deposition (carpal tunnel syndrome. bone cysts). Each person examined receives by intravenous injection approximately 1 mg of labelled β2-microglobulin (conesponding to 150 MBq of ¹²³iodine). Thyroidal iodine uptake is suppressed by administration of 0.2mg potassium iodine for 2 days prior and 1 week after the study. Following the injection of the labelled β2-microglobulin each individual is subjected to anterior and poterior whole-body imaging immediately after the injection and at 4, 24 and 48 hours using a gamma-camera. Additional images are obtained from both hands, shoulders. hips and knees or other selected areas. Using the "region-of-interest" -technique, a quantitative assessment of tracer binding is performed.

While no obvious organ localization of the injected tracer is found in normal persons in all dialysis patients the following is seen: intense specific localization of activity in amyloid deposits in the region of the carpal tunnel and bone cysts and additional minor (clinically unsuspected) amyloid deposits in other sites such as hips, knees or elsewhere in the body.

Further and more detailed examples of implementation of the invention will now be given. again by way of example only. The accompanying drawings show the results of radiodiagnostic imaging performed. as described below. in relation to four patients.

The specific details of the drawings represent the following :-
Figure 1 Dorsal (Fig. 1a) and left lateral (Fig. 1b) images of the pelvic area of patient G.A. at 48 hours after injection of the label. Two sites of increased readioactivity (marked "1" and "2") corresponded to a subcutaneous DRA tumor ("1") and to a subcutaneous site from which a DRA tumor had previously been excised ("2"). A further side (marked "3") corresponds to a radiologically proven cystic bone translucency of the femoral neck.
Figure 2 dorsal image at 72 hours after injection of the label of the shoulders/neck of patient F.R.
Figure 3 ventral image (72 hours after injection of the label) of the knees of patient F.R.
Figure 4 dorsal image at 144 hours after injection of the label of the shoulders and neck of patient R.R.

Three patients (G.A., F.R., N.D.) on chronic hemodialysis using cellulose-based membranes for 10 to 16 years were studied after written informed consent to the protocol was obtained. Injection of the radiolabelled protein was approved by the ethics committee of the Hannover Medical School. Patient G.A. presented with subcutaneous nodules overlying the ischium (biopsy proven DRA), a right-sided humeroscapular periarthritis and pelvic bone cystic translucencies. Patient F.R. presented with a left-sided humeroscapular periarthritis. He received a bilteral artifical hip replacement in 1987 (DRA proven in the femoral and synovial tissue). Patient N.D. presented with bilateral humeroscapular periarthritis and small cystic transluciencies of the fifth to seventh cervical vertebra. Cyst biopsy disclosed a DRA.

In addition, a fourth patient (R.R.), was studied, who was on regular hemodialysis treatment for 6 months only and did not show clinical or radiological evidence of DRA.

All patients were anuric and no patient had any other disease known to be associated with other types of amyloid deposition.

β₂-microglobulin was obtained from an uremic patient and prepared under sterile conditions as described above. HPLC. SDS-PAGE electrophoresis and Western blotting of the final product after lyophilisation and storage at -20°C only revealed a single peak/band. For each scan 0.1 mg β₂m was labelled with 370 MBq of Na¹³¹I (IBS.30. Amersham, Braunschweig, FRG) using the Chloramine T method. After gel filtration had been performed on a Sephadex® G 25 column (Pharmacia, Nurnberg, FRG) to remove free iodine, the labelled protein was then sterilized by passage through a 0.22 µm filter. The final preparations were tested for pyrogenicity (Pharmatox. Sehnde. FRG), sterility and content of LAL-reactive material (Dept. of Microbiology, Hannover Medical School, FRG). Paper-chromatography and HPLC of the labelled product showed a single main band/peak conesponding to 90 % (paper-chromatography) or 80% (HPLC) of the total radioactivity and immunological reactivity was confirmed by ELISA (Pharmacia, Numberg. FRG).

Two days prior to the study and during the subsequent 10 days all patients received 400 mg sodium perchlorate thrice daily to block thyroidal uptake of free iodine.

The study was initiated by intravenous injection of appropximately 20-30 µg radiolabelled β₂m, corresponding to 60-80 MBq, i.e. a radiation absorbed dose of 11-15 mSv (whole body) or 13-17 mSv (red bone marrow, testes). Images of selected areas were obtained in each patient at 48-72 hours after injection by means of a gamma-camera (ZLC 37, 360 keV collimator, Siemens, Erlangen, FRG).

Nine days after the injection of radiolabelled β₂m, the subcutaneous tumor overlying the left ischium of patient G.A. was surgically removed. Histology, immunohistology and electronmicroscopy demonstrated a DRA-tumor with concomitant amyloid infiltration of the skin. The radioactivity remaining in a specimen of the tumor was compared to the radioactivity in biopsies of the surrounding fat-, muscle- and subcutaneous tissue, which were also obtained during surgery. Following this, all tissue specimens were homogenized in PBS-buffer (pH 7.4) and protein was then precipitated by the addition of TCA (30 minutes, 4°C). After centrifugation and repeat washing with TCA, the radioactivity remaining in the pellet was determined and compared to the total counts in the homogenized tissues before TCA precipitation.

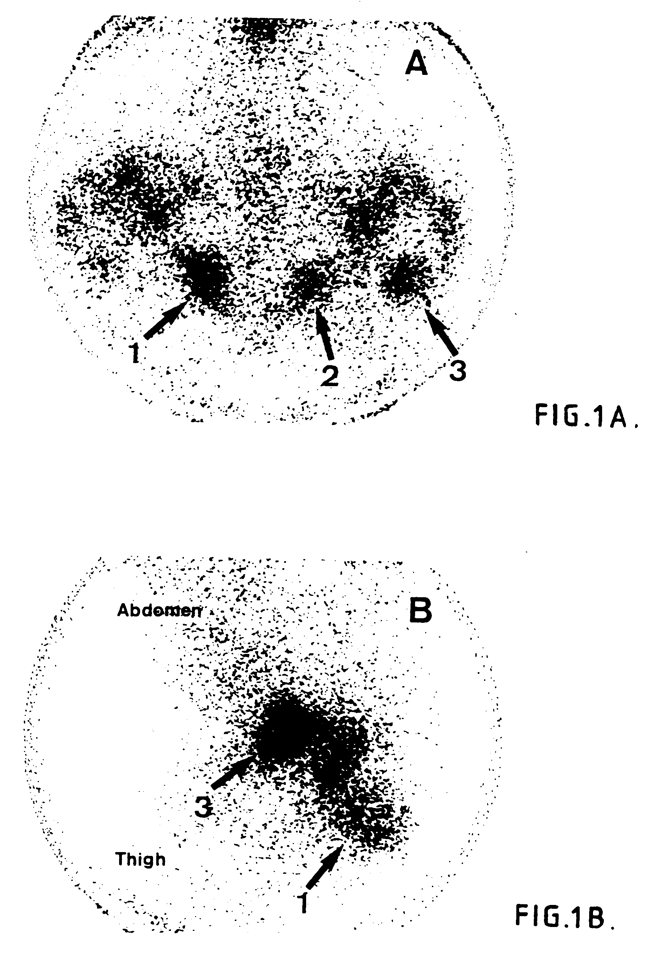

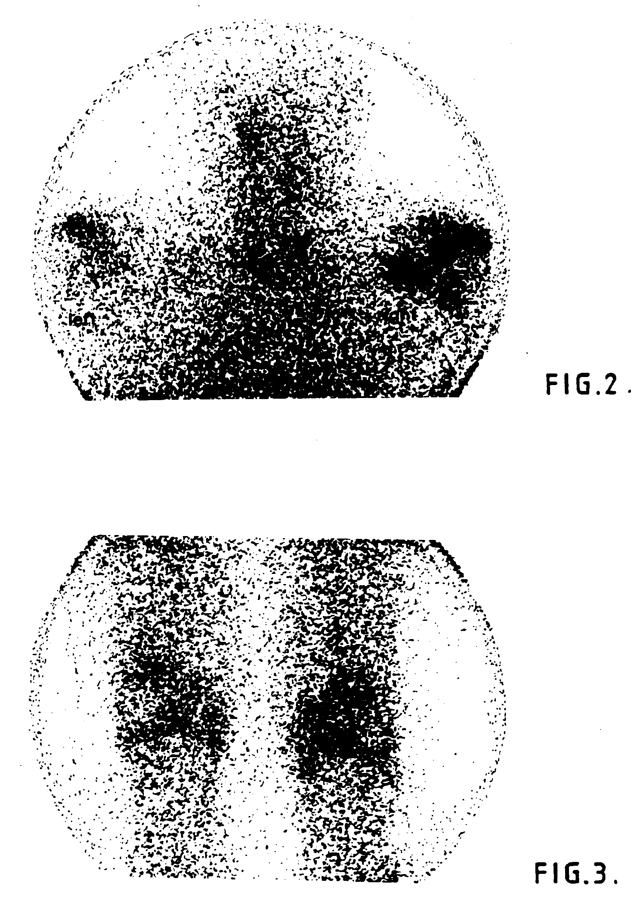

The results of the radiodiagnostic imaging were as follows :-
Patient G.A.: As shown in Figs. 1a and 1b a non-homogenous distribution of detectable radioactivity was noted in and around the pelvis during a scan at 48 hours after injection of ¹³¹I-β₂m. This pattern was constant during a repeat scan at 144 hours after injection. As indicated in the legend of Fig. 1, some sites of local enrichment of ¹³¹I-β₂m correlated with clinical or radiological findings, while for the remaining sites no clinical or radiological signs or symptoms were noted. Minor enrichment of radioactivity was noted overlying the lumbar vertebrae.
Patient F.R.: As shown in Fig. 2 accumulation of detectable radioactivity was detected during a scan at 48 hours after injection of the label in both shoulders, although clinical and radiological humeroscapular periarthritis was noted in the left shoulder only. Further sites of accumulation were both knees (fig. 3) as well as several pelvic sites in spite of negative clinical or radiological examination.
Patient N.D.: Local accumulation of ¹³¹I-β₂m was detected over both shoulders (clinical and radiological bilateral periarthritis), knees and in and around the pelvis (no clinical or radiological correlates).

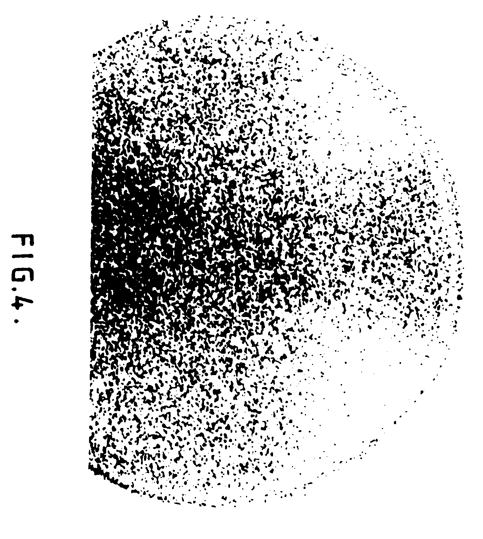

Furthermore, minor accumulation was noted over the lower cervical vertebrae corresponding to radiologically proven cystic bone translucencies of the 5th to 7th cervical vertebra.
Patient R.R.: No specific local accumulation of the label was detected during scans at 48 or 144 hours after injection of ¹³¹I-β₂m (Fig. 4).

The excised tissue of patient G.A. was also studied. The radioactivity of the tissue excised during surgery (expressed as counts/minute/mg tissue) was: muscle - 1.1, fat - 4.8, skin - 9.5, amyloid-tumor - 52.9 cpm/mg The TCA-precipitable radioactivity (expressed as percentage of the total radioactivity) in the tissues amounted to: muscle - 75.5%, fat - 82.6%, skin - 88.7%. amyloid-tumor - 92.6%.

The results of this study demonstrate that an uneven distribution of intravenously injected ¹³¹I-β₂m occurred after 48 hours in three long-term hemodialysis patients with biopsy-proven DRA. The data obtained from the excised tissue of patient G.A. showed that the uneven distribution of the injected tracer was due to specific enrichment of radioactivity in amyloid deposits of tissues with a histologically proven DRA but not in other surrounding tissues. The majority of the radioactivity in the amyloid-infiltrated tissues was precipitable by TCA suggesting that the accumulation ¹³¹iodine was bound to protein. The results therefore provide strong in-vivo evidence of a specific enrichment of the injected DRA precursor protein β₂m in the amyloid deposits of long-term hemodialysis patients. The different patterns of localization seen in the three patients strongly suggest that there was no unspecific, non-amyloid uptake of ¹³¹I-β₂m.

Local enrichment of injected β₂m may be due to direct amyloid fibril formation from the injected protein or to particularly high binding affinity of the amyloid fibrils for β₂m. Recent results have demonstrated that in addition to intact β₂m, fragments of this protein are also present in DRA, suggesting that proteolysis may take part in the pathogenesis of DRA fibril formation. A relatively strong binding of injected ¹³¹I-β₂m to the amyloid tissue with little or no reversibility is indicated by the finding of a persistent local enrichment of the label despite a marked decrease of plasma radioactivity at 6 days after injection.

The preliminary experience of this study suggests that the labelled protein is capable of detecting DRA deposits that are not associated with clincial or radiological abnormalities. The absence of specific tissue. synovial or sceletal accumulation of ¹³¹I-β₂m in patient R.R. may permit prospective studies to predict whether the progression of DRA may be altered by dialysis related therapeutic strategies.

In addition to the methods described above a description will now be given in relation to the molecular structure of injected amyloidogenic precursor molecule(s) and new diagnostic approaches derived therefrom. A description will also be given of how, at the time of the examination, to differentiate progressive from non-progressive amyloid deposits.

Further, a method will be described, which can be used to support the non-invasive diagnostic evaluation of amyloid deposits.

As described above, ¹³¹iodine-radiolabelled β2-microglobulin (and/or immunologically related proteins), derived from human uremic blood, can be used to visualize dialysis related amyloid deposits in vivo. Western blotting of the product after the labelling and/or the purification, but prior to injection demonstrates a main band at a molecular weight of 12,000 Dalton (corresponding to the molecular weight of native β2-microglobulin) as well as less intense bands at 24,000 Dalton and in the high molecular weight range. As the initial biochemical purification procedure excludes the co-purification of such higher molecular weight proteins, a spontaneous aggregation of the purified protein therefore must have occured in vitro. This is in agreement with previous findings, demonstrating that an aggregation/precipitation of β2-microglobulin occurs in vitro if the salt concentration of the surrounding solution is decreased. The mechanism involved in this is most likely the formation of hydrophobic bonds. which are stable enough to persist once the salt concentration of the surrounding solution is increased again.

Following the intravenous injection of the ¹³¹iodine-labelled protein(s) into hemodialysis patients. a partial degradation (deiodination/proteolysis) of the protein(s) occurred within 24 hours. This is indicated by the occurrence of radioactive material. which could not be precipitated with trichloroacetic acid (in contrast, the labelled protein(s) did not degrade significantly during the same time period, if they were kept in vitro at 4°C). Therefore, the specific accumulation of the label in the amyloid deposits. described above, may in part be due to the deposition of proteolytically derived, but still radioactive degradation products of the original protein(s). On the other hand, a further dimerization/polymerization of the injected protein(s) also seems to have occurred. This is demonstrated by the decreased permeability of a highly permeable hemodialysis membrane for the radiolabelled β2-microglobulin (or related proteins) as compared to endogenous circulating β2-microglobulin. Thus, desides the intact radiolabelled protein(s) and derived fragments, aggregates of variable size of the injected protein(s) may also account for the specific accumulation of radioactivity in the amyloid deposits.

Based on these experiences, molecules with a molecular weight of 24,000 Dalton have been purified (corresponding to twice the size of native β2-microglobulin) and immunological reactivity with anti-β2-microglobulin antibodies from human uremic hemofiltrate, using the methods described above. Biochemical analysis as described above, of the isolated product showed a main fraction at a molecular weight of 24,000 Dalton and some less intense higher molecular weight bands. This product was then radiolabelled, purified and sterilized as described above. A dose of 1.60 MBq of ¹³¹iodine labelled protein was injected into hemodialysis patients with a proven dialysis related amyloidosis. At 48 to 72 hours after the injection an intense accumulation of the label was observed in sites corresponding to clinically/radiologically proven amyloid deposits. Comparison of the scans using this radiolabelled protein(s) with the scans obtained previously show a higher degree of tracer-accumulation and a lesser non-specific background. In subsequent experiments it was therefore possible to reduce the dose of radioactive iodine to 40 MBq per examination.

In further experiments the 24,000 Dalton protein(s) described above were used to obtain polymers of this protein. For this the salt concentration of the surrounding solution was decreased slowly to an empirically derived point, where polymerization but not precipitation of the protein(s) had occurred. If this final polymeric product was then processed and used for in vivo scanning of amyloid deposits as described above, the same results as with the 24,000 Dalton product were obtained. In contrast however, this polymeric radiolabelled protein(s) allowed the detection of dialysis associated amyloid deposits in (pre-)hemodialysis patients with a high urinary output and in patients which had a functioning renal transplant (in these patients scanning for amyloid deposits with the low molecular weight protein(s) or the 24,000 Dalton protein(s) yielded only weak accumulations of tracer due to renal excretion of the injected radiolabelled product).

Thus, the diagnostic workup of AB2M-amyloidosis is further improved. A question of considerable clinical importance is, whether clinically or radiologically proven amyloid deposits will progress further or whether they have arrested in their expansion. The present invention may enable this question to be answered.

During the scanning with either of the products described above, it became apparent, that occasional dialysis related amyloid deposits especially those in the hands, did not accumulate the radiolabelled protein(s), while highly specific accumulation occurred elsewhere. In contrast, using the known non-invasive ¹²³iodine labelled serum amyloid P component, an imaging of dialysis associated amyloid deposits preferentially in the hands was noticed. As serum amyloid P component passively and non-specifically binds to amyloid fibrils, it will not differentiate between progressive and non-progressive deposits. If however, the radiolabelled precursor molecule is used for radiodiagnostic imaging of amyloid deposits, it will preferentially label progressive amyloid deposits as it is incorporated into amyloid fibrils after an interaction with inflammatory cells (especially macrophages), surrounding such deposits.

Besides shedding new light on the natural course of amyloid deposits the present invention may therefore allow predictions to be made concerning the outcome of certain deposits and may thus serve as a guideline for therapeutic approaches.

The present invention provides a method of detecting amyloid deposits in vivo in a specific, non-invasive way. However, the procedures described so far yield only semiquantitative assessments of the amount of amyloid present. A supplementary non-invasive method which allows a better characterization of the extent of the deposits is, therefore, beneficial. Thus, the following is a method of diagnosing and following amyloid deposits in a more quantitative manner.

Following clinical and radiological workup and radionuclide imaging with one of the proteins described above. all patients with scan proven dialysis related amyloid deposits were submitted to nuclear magnetic resonance (NMR) imaging. In the cases of localized amyloid deposits a close correlation of the scan results with those obtained by NMR was detected. By reducing the thickness of the cross-sectional images of the human body. an exact two-dimensional assessment of the maximum diameter of the deposits was obtained. Furthermore. by integration of adjacent images, an approximate estimation of the three-dimensional size of the deposits was possible.

The additional use of NMR imaging, although not specific for amyloid, may allow amyloid deposits to be monitored and quantified. Before use of NMR, it is necessary for the presence of the amyloid to be definitively established by radionuclide scanning or clinical or radiological methods of examination.

By way of example a description has thus been given of how the present invention improves the diagnostic workup of AB2M-amyloidosis. Repeated examination will enable clarification of whether any therapeutic regime is able to halt or reverse the progression of these amyloid-deposits. As described, ¹²³iodine and ¹³¹iodine has been used to label the β2-microglobulin but ^{99m}TC can also be used, or any other suitable isotope, which has a short half-life and which can easily be coupled to proteins. The isotope should be a gamma emitter since use is preferably made of a gamma camera. Furthermore, the natural occurring protein β2-microglobulin has been used, but a recombinant form of β2-microglobulin could be used, or a synthetic peptide with epitopes that would bind to the amyloid fibrillar ligands after similar radio labelling. This invention is not confined to the amyloidosis of dialysis patients. but can be applied in a similar fashion to any other specific or mixture of amyloid fibrillar deposited protein syndromes.

The use of the highly specific amyloidogenic precursor molecule enables physicians to couple it to the therapeutic agents (e.g. agents altering the microenvironment surrounding the amyloid fibrills or agents that promote destruction and resolution of amyloid fibrills) and thereby for the first time offer specific anti-amyloid treatment to the patient.

## Claims

1. Radiolabelled β2-microglobulin, or a naturally occurring variant thereof, for use in the diagnosis and treatment of amyloidosis.

2. A radiolabelled product as claimed in claim 1, wherein the said β2-microglubin is naturally or synthetically derived.

3. A radiolabelled product according to claim 1 or 2, wherein ¹²³I is used to label the product.

4. A radiolabelled product as claimed in claim 1 or 2, wherein ¹³¹iodine is used to label the product.

5. A radiolabelled product as claimed in claim 1 or 2, wherein ^{99m}TC is used to label the product.

6. A radiolabelled product according to any preceding claim, for use in the treatment or diagnosis of progressive amyloidosis.

## Patentansprüche

1. Radioaktiv markiertes β2-Mikroglobulin, oder eine natürlich vorkommende Variante davon, zur Verwendung in der Diagnose und Behandlung von Amyloidose.

2. Radioaktiv markiertes Produkt nach Anspruch 1, dadurch gekennzeichnet, daß das β2-Mikroglobulin natürlich oder synthetisch erhalten wurde.

3. Radioaktiv markiertes Produkt nach Anspruch 1 oder 2, dadurch gekennzeichnet, daß ¹²³I zur Markierung des Produkts verwendet wurde.

4. Radioktiv markiertes Produkt nach Anspruch 1 oder 2, dadurch gekennzeichnet, daß ¹³¹I zur Markierung des Produkts verwendet wurde.

5. Radioaktiv markiertes Produkt nach Anspruch 1 oder 2, dadurch gekennzeichnet, daß ^{99m}Tc zur Markierung des Produkts verwendet wurde.

6. Radioaktiv markiertes Produkt nach einem der vorherigen Ansprüche zur Verwendung in der Behandlung oder Diagnose von fortgeschrittener Amyloidose.

## Revendications

1. Une microglobuline B2 radiomarquée, ou une variante correspondante de production naturelle, destinée à être utilisée dans le diagnostic et le traitement de l'amylose.

2. Une substance radiomarquée selon la revendication 1, dans laquelle ladite microglobuline B2 est obtenue naturellement ou synthétiquement.

3. Une substance radiomarquée selon les revendications 1 ou 2, dans lequel du ¹²³iode est utilisé pour marquer la substance.

4. Une substance radiomarquée selon les revendications 1 ou 2, dans laquelle ¹³¹iode est utilisé pour marquer la substance.

5. Une substance radiomarquée selon les revendications 1 ou 2, dans laquelle du ^{99m}TC est utilisé pour marquer la substance.

6. Une substance radiomarquée selon l'une quelconque des revendications précédentes servant au traitement ou au diagnostic de l'amylose évolutive.
